(19) Europäisches Patentamt
European Patent Office
Office européen des brevets

(11) Veröffentlichungsnummer: **0 167 893**
B1

(12) # EUROPÄISCHE PATENTSCHRIFT

(45) Veröffentlichungstag der Patentschrift:
**14.09.88**

(21) Anmeldenummer: **85107543.2**

(22) Anmeldetag: **19.06.85**

(51) Int. Cl.⁴: **C 07 F 9/65** // C07D239/36, C07D239/46, C07D239/52, C07D239/54

(54) **Verfahren zur Herstellung von Phosphorsäurederivaten und Zwischenprodukten.**

(30) Priorität: **27.06.84 DE 3423622**

(43) Veröffentlichungstag der Anmeldung:
**15.01.86 Patentblatt 86/3**

(45) Bekanntmachung des Hinweises auf die Patenterteilung:
**14.09.88 Patentblatt 88/37**

(84) Benannte Vertragsstaaten:
**BE CH DE FR GB IT LI NL**

(56) Entgegenhaltungen:
**FR - A - 2 365 577**

**JOURNAL OF THE CHEMICAL SOCIETY, Dezember 1965, Seiten 7116-7119; D.T. HURST u.a.: "Pyrimidines. Part XIV. Synthesis of 2,5-Dihydroxy-pyrimidine"**
**CHEMICAL ABSTRACTS, Band 86, Nr. 21, 23 Mai 1977, Seite 440, Nr. 155305c, Columbus, Ohio, US; H. KAEMMERER u.a.: "Halogen as a readily cleavable protective group for reactive positions in phenols and phenolic compounds", & ORG. PREP. PROCED. INT. 1976, 8(6), 245-8**
**D.J. Brown, The Pyrimidines, Supplement I, 1970, *Wiley-Interscience*, pp. 148, 149**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**

**EP 0 167 893 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von insektiziden Pyrimidinylphosphorsäurederivaten sowie von Zwischenprodukten, welche für die Durchführung des Verfahrens verwendet werden können.

Es ist bereits bekannt, dass man bestimmte pestizide Phosphorsäurepyrimidinester erhält, wenn man entsprechende Phosphorsäureesterchloride mit 5-Hydroxypyrimidinen umsetzt (vgl. DE-OS 2 643 262 und DE-OS 2 706 127). Diese Herstellungsmethode ist jedoch wegen des Fehlens geeigneter Ausgangsverbindungen bzw. wegen unbefriedigender Herstellungsmethoden hierfür nur begrenzt anwendbar. Es besteht daher Bedarf an neuen Herstellungsverfahren für Phosphorsäurepyrimidinester und zugehörigen Zwischenprodukte.

Es wurde nun gefunden, dass man die Verbindungen der allgemeinen Formel (I)

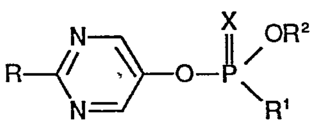

(I)

in welcher
R für Wasserstoff, Alkoxy, Alkylamino, Dialkylamino oder für gegebenenfalls substituierte Reste aus der Reihe Alkyl, Cycloalkyl und Aryl steht,
$R^1$ für gegebenenfalls substituierte Reste aus der Reihe Alkyl, Alkoxy, Alkylthio, Mono- oder Dialkylamino und Phenyl steht,
$R^2$ für gegebenenfalls substituiertes Alkyl steht und
X für Sauerstoff oder Schwefel steht, erhält, wenn man

a) Verbindungen der allgemeinen Formel (II)

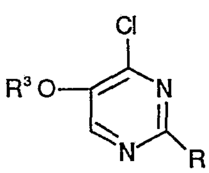

(II)

in welcher
R die oben angegebene Bedeutung hat, und
$R^3$ für gegebenenfalls substituiertes Benzyl steht, mit Wasserstoff in Gegenwart von Hydrierungskatalysatoren, in Gegenwart von Säureakzeptoren und in Gegenwart von Verdünnungsmitteln, bei Temperaturen zwischen 20°C und 150°C zu den Verbindungen der allgemeinen Formel (III)

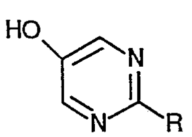

(III)

in welcher
R die oben angegebene Bedeutung hat,
umsetzt und anschliessend

b) die Verbindungen der allgemeinen Formel (III), gegebenenfalls nach ihrer Isolierung, mit Verbindungen der allgemeinen Formel (IV)

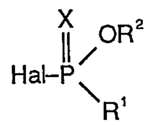

(IV)

in welcher
Hal für Halogen steht und
X, $R^1$ und $R^2$ die oben angegebene Bedeutung haben, gegebenenfalls in Gegenwart eines Lösungsmittels, umsetzt und die Verbindungen der allgemeinen Formel (I) isoliert.

Gemäss diesen Verfahren ist es möglich, die Verbindungen der Formel (I) auf einfache Weise in guter Reinheit und Ausbeute herzustellen. Das Verfahren ist im Hinblick auf die Art der gewünschten Substituenten sehr breit anwendbar. Weiterhin sind die als Zwischenprodukte einzusetzenden Verbindungen stabil und können gut gelagert und gehandhabt werden.

Bevorzugte Substituenten bzw. Bereiche der in den oben und nachfolgend erwähnten Formeln aufgeführten Reste werden im folgenden erläutert:

Alkoxy R steht für geradkettiges oder verzweigtes Alkoxy mit vorzugsweise 1 bis 12, insbesondere 1 bis 6 und besonders bevorzugt 1 bis 4 Kohlenstoffatomen. Beispielhaft seien Methoxy, Ethoxy, n-Propoxy, i-Propoxy, n-Butoxy, i-Butoxy, sec.-Butoxy und tert.-Butoxy genannt.

Mono- oder Di-Alkylamino R steht für eine Aminogruppe mit 1 oder 2 Alkylgruppen, vorzugsweise 2 Alkylgruppen, welche jeweils geradkettig oder verzweigt sein können und vorzugsweise 1 bis 5, insbesondere 1 bis 3 Kohlenstoffatome enthalten, wobei Methyl, Ethyl, n- und i-Propyl genannt seien. Beispielhaft seien Dimethylamino, Diethylamino, Di-n-propylamino und Di-i-propylamino aufgeführt.

Als gegebenenfalls substituiertes Alkyl R steht geradkettiges oder verzweigtes Alkyl mit 1 bis 20, vorzugsweise 1 bis 12, insbesondere 1 bis 6 und besonders bevorzugt 1 bis 4 Kohlenstoffatomen. Beispielhaft seien gegebenenfalls substituiertes Methyl, Ethyl, n-Propyl, i-Propyl, n-Butyl, sec.-Butyl, i-Butyl, tert.-Butyl, n-Pentyl, i-Pentyl und tert.-Pentyl genannt.

Als gegebenenfalls substituiertes Cycloalkyl R steht Cycloalkyl mit vorzugsweise 3 bis 8, insbesondere 3, 5 oder 6 Kohlenstoffatomen. Beispielhaft seien gegebenenfalls substituiertes Cyclopropyl, Cyclobutyl, Cyclopentyl, Cyclohexyl und Cycloheptyl genannt.

Als gegebenenfalls substituiertes Aryl R steht Aryl mit vorzugsweise 6 bis 10 Kohlenstoffatomen im Arylteil. Beispielhaft seien gegebenenfalls substituiertes Phenyl oder Naphthyl, insbesondere Phenyl genannt.

Die in der Definition von R genannten substituierten Reste sowie der Phenylring im Benzylrest $R^3$ können einen oder mehrere, vorzugsweise 1 bis 3, insbesondere 1 oder 2 gleiche oder verschiedene Substituenten tragen. Als Substituenten für Alkyl, Cycloalkyl und Aryl und Benzyl seien beispielhaft aufgeführt:

Alkoxy und Alkylsulfonyl mit 1 bis 4 Kohlenstoffatomen, wie Methoxy, Ethoxy, n-Propoxy, i-Propoxy, n-Butoxy, i-Butoxy, sec.-Butoxy, tert.-Butoxy, Methylsulfonyl, Ethylsulfonyl, n-Propylsulfonyl, i-Propylsulfonyl, n-Butylsulfonyl, i-Butylsulfonyl und tert.-Butylsulfonyl.

Als Arylsubstituenten, Cycloalkylsubstituenten und als Substituenten im Phenylring der Benzylgruppe kommen ausserdem noch $C_1$-$C_4$-Alkyle in Frage, wie Methyl, Ethyl, n-Propyl, i-Propyl, n-Butyl, i-Butyl, sec.-Butyl und tert.-Butyl.

Vorzugsweise steht

R für Wasserstoff, für Alkoxy mit 1 bis 12 Kohlenstoffatomen, für Mono- oder Di-Alkylamino mit jeweils 1 bis 6 Kohlenstoffatomen im Alkylteil, für gegebenenfalls durch $C_1$-$C_4$-Alkoxy oder $C_1$-$C_4$-Alkylsulfonyl substituiertes Alkyl mit 1 bis 12 Kohlenstoffatomen, für gegebenenfalls durch $C_1$-$C_4$-Alkyl substituiertes Cycloalkyl mit 3 bis 8 Kohlenstoffatomen und für gegebenenfalls durch $C_1$-$C_4$-Alkyl, $C_1$-$C_4$-Alkoxy oder $C_1$-$C_4$-Alkylsulfonyl substituiertes Aryl mit 6 bis 10 Kohlenstoffatomen.

Besonders bevorzugt steht

R für Wasserstoff, Alkoxy mit 1 bis 6 Kohlenstoffatomen, Mono- oder Dialkylamino mit jeweils 1 bis 4 Kohlenstoffatomen im Alkylteil oder für gegebenenfalls durch Methoxy, Ethoxy, Methylsulfonyl oder Ethylsulfonyl substituiertes Alkyl mit 1 bis 6 Kohlenstoffatomen, für gegebenenfalls durch Methyl oder Ethyl substituiertes Cycloalkyl mit 3 bis 6 Kohlenstoffatomen und für gegebenenfalls durch Methyl, Ethyl, Methoxy, Ethoxy, Methylsulfonyl oder Ethylsulfonyl substituiertes Phenyl.

Ganz besonders bevorzugt steht R für Methyl, Isopropyl und tert.-Butyl.

Die gegebenenfalls substituierten Alkylgruppen $R^1$ und $R^2$ enthalten vorzugsweise 1 bis 6, insbesondere 1 bis 4 und besonders bevorzugt 1 oder 2 Kohlenstoffatome. Beispielhaft seien Methyl, Ethyl, n- und i-Propyl, n-, i-, s- und t-Butyl genannt.

Die Alkylgruppen der gegebenenfalls substituierten Alkyl- und Dialkylaminogruppen $R^1$ haben vorzugsweise die für Alkylgruppen $R^1$ und $R^2$ oben angegebene vorzugsweise Bedeutung. Beispielhaft seien Methyl-, Ethyl-, n- und i-Propylamino sowie Dimethyl-, Diethyl- und Methyl-ethylamino aufgeführt.

Die Alkoxy- und Alkylthioreste $R^1$ enthalten vorzugsweise 1 bis 6, insbesondere 1 bis 4 und besonders bevorzugt 1 oder 2 Kohlenstoffatome. Beispielhaft seien Methoxy, Ethoxy, n- und i-Propoxy sowie Methylthio, Ethylthio und n- und i-Propylthio genannt.

Die gegebenenfalls substituierten Reste $R^1$ und $R^2$ können einen oder mehrere, vorzugsweise 1 bis 3, insbesondere 1 oder 2 gleiche oder verschiedene Substituenten tragen. Als Substituenten seien beispielhaft aufgeführt: Alkyl (gilt nicht für die Fälle in welchen $R^1$ und $R^2$ für Alkyl steht) mit vorzugsweise 1 bis 4, insbesondere 1 oder 2 Kohlenstoffatomen, wie Methyl, Ethyl, n- und i-Propyl und n-, i-, s- und t-Butyl; Alkoxy mit vorzugsweise 1 bis 4, insbesondere 1 oder 2 Kohlenstoffatomen, wie Methoxy, Ethoxy, n- und i-Propoxy und n-, i-, s- und t-Butyloxy; Alkylthio mit vorzugsweise 1 bis 4, insbesondere 1 oder 2 Kohlenstoffatomen, wie Methylthio, Ethylthio, n- und i-Propylthio und n-, i-, s- und t-Butylthio; Halogen, vorzugsweise Fluor, Chlor, Brom und Jod, insbesondere Chlor und Brom; Cyano und Nitro.

Hal in der allgemeinen Formel (IV) steht für Fluor, Chlor, Brom und Jod, vorzugsweise für Fluor, Chlor und Brom, insbesondere für Chlor.

Die in der Verfahrensstufe a) einzusetzenden Verbindungen der Formel (II) sind bekannt und/ oder lassen sich nach bekannten Methoden herstellen, indem man z.B. Verbindungen der allgemeinen Formel (V)

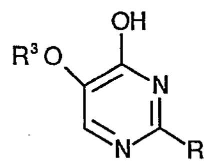

(V)

in welcher

R und $R^3$ die oben angegebenen Bedeutungen haben, in Gegenwart von Halogenierungsmitteln, wie z.B. Phosphoroxychlorid, Phosphortrichlorid, Oxalylchlorid, Phosgen oder Thionylchlorid, und Verdünnungsmitteln, wie z.B. Dimethylformamid oder Chloroform, bei Temperaturen zwischen 0°C und 70°C umsetzt (vgl. Aust. J. Chem. 29 (6), 1335 ff, J. Chem. Soc. 1960, S. 4594 und die Herstellungsbeispiele).

Als Beispiele für erfindungsgemäss verwendbare Verbindungen der Formel (II) seien aufgeführt:

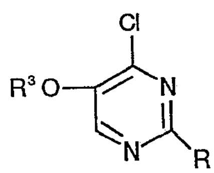

(II)

Tabelle 1

| R | $R^3$ |
|---|---|
| H | 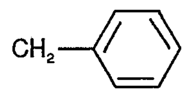 |
| $CH_3$ | 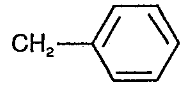 |
| $C_2H_5$ | 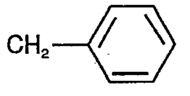 |
| n-$C_3H_7$ | 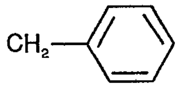 |

Tabelle 1 (Fortsetzung)

| R | $R^3$ |
|---|---|
| $i\text{-}C_3H_7$ | 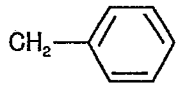 |
| $n\text{-}C_4H_9$ | 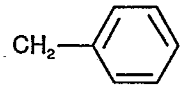 |
| $i\text{-}C_4H_9$ | 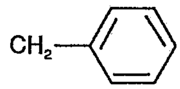 |
| sec.-$C_4H_9$ | 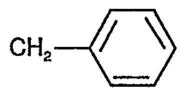 |
| tert.-$C_4H_9$ | 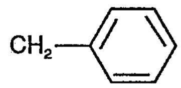 |
| tert.-$C_5H_{11}$ (neopentyl) | 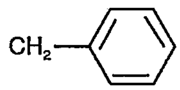 |
| 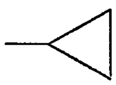 | 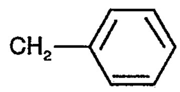 |
| 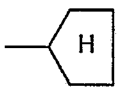 | 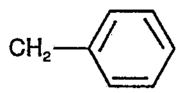 |
| 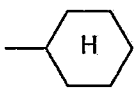 | 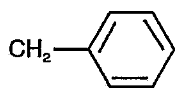 |
| $OCH_3$ | 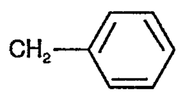 |
| $OC_2H_5$ | 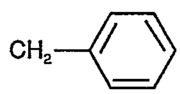 |
| $OC_3H_7\text{-}n$ | 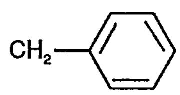 |
| $OC_3H_7\text{-}i$ | 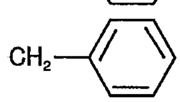 |
| $OC_4H_9\text{-}n$ | 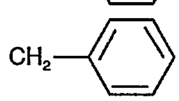 |
| $OC_4H_9\text{-}i$ | 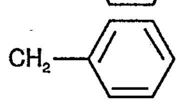 |
| $OC_4H_9$-sec. | 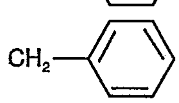 |
| $OC_4H_9$-tert. | 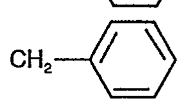 |
| $NH\text{-}CH_3$ | 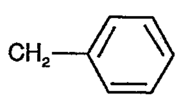 |
| $NH\text{-}C_2H_5$ | 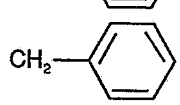 |

Tabelle 1 (Fortsetzung)

| R | $R^3$ |
|---|---|
| $NH\text{-}C_3H_7\text{-}n$ | 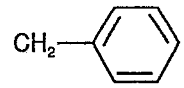 |
| $NH\text{-}C_3H_7\text{-}i$ | 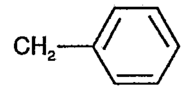 |
| $NH\text{-}C_4H_9\text{-}n$ | 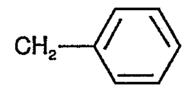 |
| $N(CH_3)_2$ | 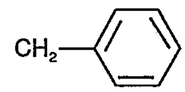 |
| $N(C_2H_5)_2$ | 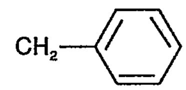 |
| $N(C_3H_7\text{-}n)_2$ | 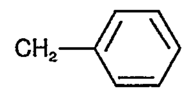 |
| $N(C_3H_7\text{-}i)_2$ | 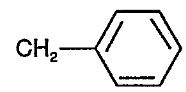 |
| $N(C_4H_9\text{-}n)_2$ | 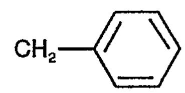 |
| 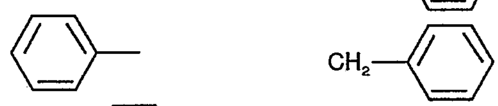 | |
| 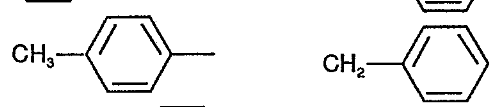 | |
| 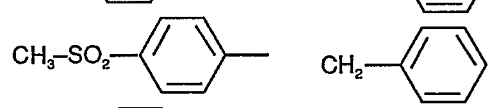 | |
| 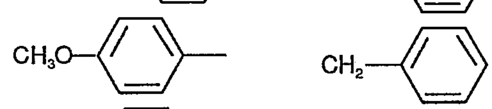 | |
| 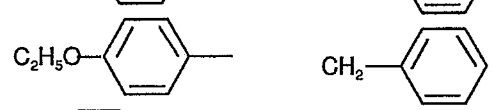 | |
| 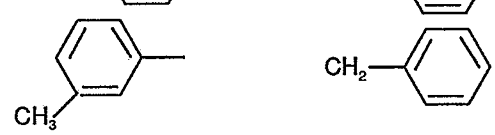 | |
| 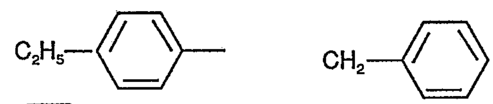 | |
| 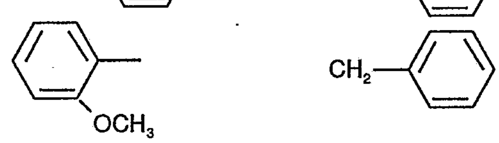 | |
| H | 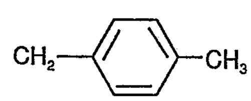 |
| $CH_3$ | 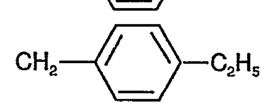 |

Tabelle 1 (Fortsetzung)

| R | $R^3$ |
|---|---|
| $CH_3$ | 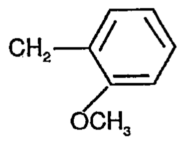 |
| $C_2H_5$ | 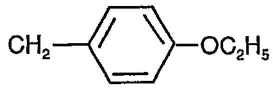 |
| $C_3H_7$-i | 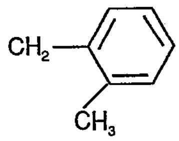 |
| $C_4H_9$-i | 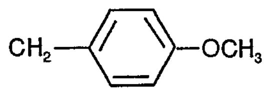 |
| $C_4H_9$-tert. | 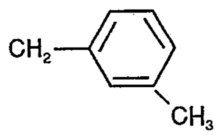 |
| $CH_2CH_2$-$OCH_3$ | 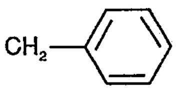 |
| $CH_2OCH_3$ | 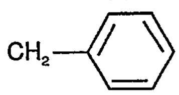 |
| $CH_2CH_2$-$OC_2H_5$ | 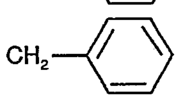 |
| $CH_2$-$SO_2$-$CH_3$ | 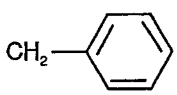 |
| $CH_2$-$CH_2SO_2CH_3$ | 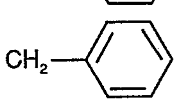 |

Im Verfahrensschritt a) werden die Verbindungen der allgemeinen Formel (II) in die Verbindungen der allgemeinen Formel (III) umgewandelt. Das Verfahren zur Herstellung der Verbindungen der allgemeinen Formel (III) gemäss Verfahrensschritt a) ist Teil der vorliegenden Erfindung.

Es ist bereits bekannt, dass man 5-Hydroxy-pyrimidine erhält, wenn man 5-Methoxy-pyrimidine in Autoklaven, bei Temperaturen zwischen 180°C und 200°C unter basischen Bedingungen umsetzt (vgl. z.B. DE-OS 2 643 262 und Coll. Czech. Chem. Comm. 40, 1078 ff (1975)). Die Nachteile dieser Verfahren bestehen darin, dass die Ausbeuten und die Reinheit der Reaktionsprodukte häufig unbefriedigend sind und ausserdem extreme Reaktionsbedingungen erforderlich sind.

Es ist ausserdem bekannt, dass man die 5-Hydroxy-pyrimidine auch in Gegenwart von Alkalihydroxiden und Glykol aus 5-Methoxy-pyrimidinen herstellen kann. Bei diesem Verfahren sind Temperaturen von ca. 200°C erforderlich. Weitere Nachteile sind die aufwendige Aufarbeitung der Endprodukte und die mässigen Ausbeuten (vgl. z.B. J. Chem. Soc. 1960, 4590 ff und Chem. Ber. 95, 803 ff (1962)). Ausserdem verlangt die Arbeitsweise in polaren hochsiedenden Lösungsmitteln wie Glykol besondere Anstrengungen zur Abwasserreinigung.

In C. R. Noller, Lehrbuch der Organischen Chemie, Seite 556, Berlin-Göttingen-Heidelberg, 1960, wird allgemein auf die leichte Spaltung der Benzyl-Sauerstoff-Bindung hingewiesen. In Chem. Abstracts, 86: 155305c (1977) wird die Abspaltung von Chlor bei einer bestimmten Phenolverbindung durch katalytische Hydrierung beschrieben. Eine mögliche Anwendung solcher Verfahren bei Pyrimidinderivaten ist hieraus jedoch nicht ableitbar.

Es wurde nun gefunden, dass man 5-Hydroxypyrimidine der Formel (III)

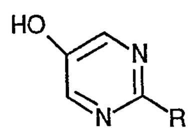

(III)

in welcher

R die oben angegebene Bedeutung hat, erhält, wenn man substituierte 4-Chlor-pyrimidin-Derivate der Formel (II)

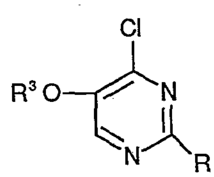

(II)

in welcher

R und $R^3$ die oben angegebenen Bedeutungen haben, mit Wasserstoff in Gegenwart von Hydrierungskatalysatoren, in Gegenwart von Säureakzeptoren und in Gegenwart von Verdünnungsmitteln bei Temperaturen zwischen 20°C und 150°C umsetzt.

Überraschenderweise ist es möglich mit Hilfe des erfindungsgemässen Verfahrens unter relativ milden Bedingungen die 5-Hydroxy-pyrimidine in guter Ausbeute und sehr hoher Reinheit zu erhalten. Weitere Vorteile des Verfahrens sind die mögliche Rückgewinnung der Katalysatoren und die Verwendung von preisgünstigen und umweltfreundlicheren Verdünnungsmitteln. Verwendet man beispielsweise für das erfindungsgemässe Verfahren 5-Benzyloxy-4-chlor-2-phenyl-pyrimidin und ein Palladium/Kohle-Gemisch als Katalysator, so kann die Reaktion durch das folgende Formelschema skizziert werden:

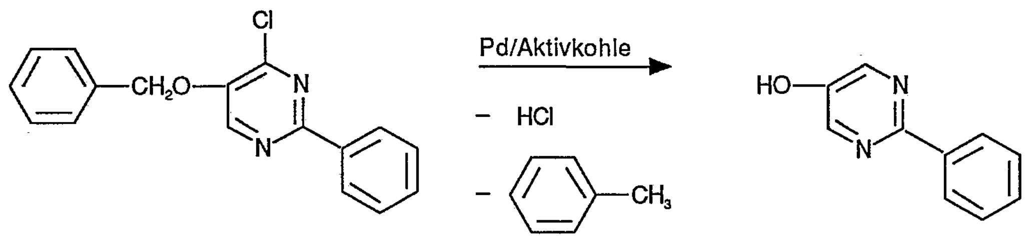


Für die Herstellung der Verbindungen der allgemeinen Formel (III) aus den Verbindungen der allgemeinen Formel (II) werden als Verdünnungsmittel niedrige aliphatische Alkohole wie z.B. Methanol, Ethanol, n- und i-Propanol, n-, i-, s- und t-Butanol oder Zweiphasensysteme aus Wasser und mit Wasser nicht mischbaren Lösungsmitteln, wie z.B. Hexan, Heptan, Cyclohexan, Methylcyclohexan, Diethylether, Toluol und Xylol verwendet.

Als Säureakzeptoren kommen für das erfindungsgemässe Verfahren alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; sowie niedere tertiäre Alkylamine, Cycloalkylamine und Aralkylamine, wie insbesondere Triethylamin.

Das erfindungsgemässe Verfahren wird in Gegenwart eines Hydrierungskatalysators durchgeführt. Es werden vorzugsweise neutrale Metallkatalysatoren wie Raney-Nickel, Raney-Cobalt oder Palladium gegebenenfalls auf üblichen Trägermaterialien, wie z.B. Aktivkohle, eingesetzt.

Die Reaktionstemperaturen können bei der Durchführung des erfindungsgemässen Verfahrens in einem grösseren Bereich variiert werden. Im allgemeinen arbeitet man zwischen 20°C und 150°C, vorzugsweise zwischen 20°C und 100°C, insbesondere zwischen 40°C und 80°C.

Das erfindungsgemässe Verfahren wird im allgemeinen bei erhöhtem Druck, vorzugsweise zwischen 5 und 60 bar, insbesondere zwischen 7 und 40 bar, durchgeführt.

Zur Durchführung des erfindungsgemässen Verfahrens setzt man auf 1 Mol 4-Chlor-pyrimidin-Derivat der Formel (II) zwischen 1 und 5 Mol, vorzugsweise zwischen 1, 2 und 3 Mol Säureakzeptor und zwischen 1 und 100 g, vorzugsweise zwischen 5 und 50 g Katalysator ein.

Die Ausgangsstoffe der Formel (II), der Säureakzeptor, der Katalysator und das Verdünnungsmittel, werden vermischt und während des Aufheizens auf die erforderliche Temperatur wird Wasserstoff eingedrückt. Bei konstanter Temperatur wird so lange Wasserstoff eingedrückt, bis die Druckkonstanz das Reaktionsende anzeigt.

Als Beispiele für die Verbindungen der Formel (III) seien die folgenden Verbindungen genannt:

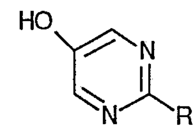

(III)

Tabelle 2

| R | R | R |
|---|---|---|
| H | | $-CH_2CH_2OCH_3$ |
| $CH_3$ | | $-CH_2OC_2H_5$ |
| $C_2H_5$ | | $-CH_2CH_2OC_2H_5$ |
| $C_3H_7$-n | | $-CH_2SO_2CH_3$ |
| $C_3H_7$-i | | $-CH_2CH_2SO_2CH_3$ |
| $C_4H_9$-sec. | | $-CH_2CH_2SO_2C_2H_5$ |
| $C_4H_9$-tert. | | $-N(CH_3)_2$ |
| $C_5H_{11}$-n | | $-N(C_2H_5)_2$ |
| $C_5H_{11}$-tert. (neopentyl) | | 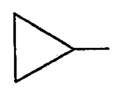 |
| $OCH_3$ | | 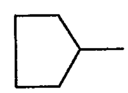 |
| $OC_2H_5$ | | 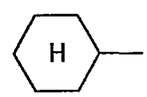 |
| $OC_3H_7$-n | 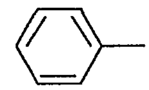 | $-CH_2OCH_3$ |
| $OC_3H_7$-i | 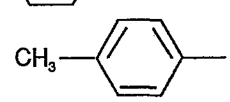 | |

In der Verfahrensstufe b) werden die Verbindungen der allgemeinen Formel (I) durch die Umsetzung der Verbindungen der allgemeinen Formeln (III) und (IV) erhalten.

Verwendet man in der Verfahrensstufe b) beispielsweise O-Ethyl-O-isopropyl-thionophosphorsäurediesterchlorid und 5-Hydroxy-2-phenyl-pyrimidin als Ausgangsstoffe, so kann die entsprechende Reaktion durch das folgende Formelschema skizziert werden:

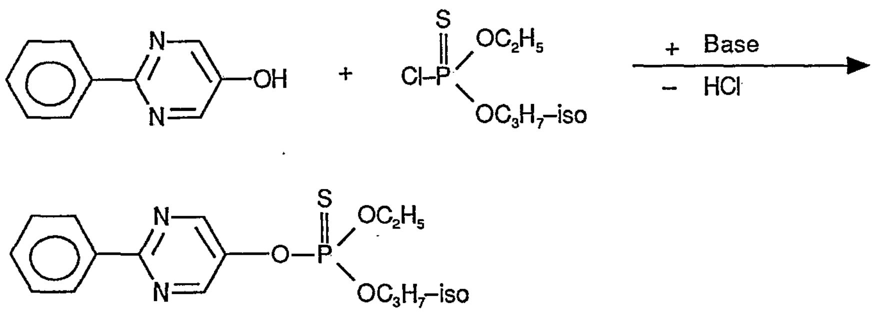


Die in der Verfahrensstufe b) einzusetzenden Ausgangsstoffe der allgemeinen Formel (IV) sind bekannt und nach literaturbekannten Verfahren und Methoden technisch gut herstellbar. Als Beispiele dafür seien im einzelnen genannt:

O,O-Dimethyl-, O,O-Diethyl-, O,O-Di-n-propyl-, O,O-Diiso-propyl-, O,O-Di-n-butyl-, O,O-Di-iso-butyl-, O,O-Di-sec.-butyl-, O-Methyl-O-ethyl-, O-Methyl-O-n-propyl-, O-Methyl-O-iso-propyl-, O-Methyl-O-n-butyl-, O-Methyl-O-iso-butyl-, O-Methyl-O-sec.-butyl-, O-Ethyl-O-n-propyl-, O-Ethyl-O-iso-propyl-, O-Ethyl-O-n-butyl-, O-Ethyl-O-sec.-butyl-, O-Ethyl-O-iso-butyl-, O-n-Propyl-O-butyl- bzw. O-iso-propyl-O-butylphosphorsäurediesterchlorid und die entsprechenden Thionoanalogen, ferner O,S-Dimethyl-, O,S-Diethyl-, O,S-Di-n-propyl-, O,S-Di-iso-propyl-, O,S-Di-n-butyl-, O,S-Di-iso-butyl-, O-Ethyl-S-n-propyl-, O-Ethyl-S-iso-propyl-, O-Ethyl-S-n-butyl-, O-Ethyl-S-sec.-butyl-, O-n-Propyl-S-ethyl-, O-n-Propyl-S-iso-propyl-, O-n-Butyl-S-n-propyl- und O-sec.-Butyl-S-ethylthiol-phosphorsäurediesterchlorid und die entsprechenden Thioanalogen, ferner O-Methyl-, O-Ethyl-, O-n-Propyl-, O-iso-Propyl-, O-n-Butyl-, O-iso-Butyl- bzw. O-sec.-Butyl-methan- bzw. -ethan-, -n-propan-, -iso-propan-, -n-butan-, -iso-butan-, -sec.-butan- bzw. -phenyl-phopshonsäureesterchlorid und die entsprechenden Thionoanalogen und O-Methyl-N-methyl-, O-Methyl-N-ethyl-, O-Methyl-N-n-propyl-, O-Methyl-N-iso-propyl-, O-Ethyl-N-methyl-, O-Ethyl-N-ethyl-, O-Ethyl-N-n-propyl-, O-Ethyl-N-iso-propyl-, O-n-Propyl-N-methyl-, O-n-Propyl-N-ethyl-, O-n-Propyl-N-n-propyl-, O-n-Propyl-N-iso-propyl-, O-iso-Propyl-N-methyl-, O-iso-Propyl-N-ethyl-, O-iso-Propyl-N-n-propyl-, O-iso-Propyl-N-iso-propyl-, O-n-Butyl-N-methyl-, O-n-Butyl-N-ethyl-, O-n-Butyl-N-n-propyl-, O-n-Butyl-N-isopropyl-, O-iso-Butyl-N-methyl-, O-iso-Butyl-N-ethyl-, O-iso-Butyl-N-n-propyl-, O-iso-Butyl-N-iso-propyl-, O-sec.-Butyl-N-methyl-, O-sec.-Butyl-N-ethyl-, O-sec.-Butyl-N-n-propyl- und O-sec.-Butyl-N-iso-propyl-phosphorsäure-monoesteramidchlorid und die entsprechenden Thionoanalogen.

Die Verfahrensstufe b) zur Herstellung der Verbindungen der allgemeinen Formel (I) wird bevorzugt unter Mitverwendung geeigneter Lösungs- und Verdünnungsmittel durchgeführt. Als solche kommen praktisch alle inerten organischen Solventien in Frage. Hierzu gehören insbesondere aliphatische und aromatische, gegebenenfalls chlorierte Kohlenwasserstoffe, wie Benzol, Toluol, Xylol, Benzin, Methylenchlorid, Chloroform, Tetrachlorkohlenstoff, Chlorbenzol oder Ether, wie Diethyl- und Dibutylether, Dioxan, ferner Ketone, beispielsweise Aceton, Methylethyl-, Methylisopropyl- und Methylisobutylketon, ausserdem Nitrile, wie Aceto- und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Kalium-tert.-butylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann innerhalb eines grösseren Bereichs variiert werden. Im allgemeinen arbeitet man zwischen 0 und 100°C, vorzugsweise bei 20 bis 60°C.

Die Umsetzung lässt man im allgemeinen bei Normaldruck ablaufen.

Zur Durchführung der Verfahrensstufe b) setzt man die Ausgangsmaterialien meist in äquivalentem Verhältnis ein. Ein Überschuss der einen oder anderen Komponente bringt keine wesentlichen Vorteile. Die Reaktionspartner werden meist in einem der oben angeführten Lösungsmittel in Gegenwart eines Säurebindemittels vereinigt und bei erhöhter Temperatur zur Vervollständigung der Reaktion eine oder mehrere Stunden gerührt. Danach versetzt man die Mischung mit einem organischen Lösungsmittel, z.B. Toluol, und arbeitet die organische Phase in üblicher Weise durch Waschen, Trocknen und Abdestillieren des Lösungsmittels auf.

Die Verbindungen der allgemeinen Formel (I) fallen meist in Form von Ölen an, die sich häufig nicht unzersetzt destillieren lassen, jedoch durch sogenanntes «Andestillieren», d.h. durch längeres Erhitzen unter vermindertem Druck auf mässig erhöhte Temperaturen von den letzten flüchtigen Anteilen befreit und auf diese Weise gereinigt werden. Zu ihrer Charakterisierung dient der Brechungsindex.

Wie bereits mehrfach erwähnt, zeichnen sich

die erfindungsgemäss erhältlichen Verbindungen der allgemeinen Formel (I) durch eine hervorragende insektizide, akarizide und nematizide Wirkung aus. Sie wirken gegen Pflanzen-, Hygiene- und Vorratsschädlinge und auf dem veterinärmedizinischen Sektor. Sie besitzen bei geringer Phytotoxizität sowohl eine gute Wirkung gegen saugende als auch fressende Insekten und Milben.

Aus diesem Grunde können die erfindungsgemäss erhältlichen Verbindungen der allgemeinen Formel (I) mit Erfolg im Pflanzenschutz sowie auf dem Hygiene-, Vorratsschutz- und Veterinärsektor als Schädlingsbekämpfungsmittel eingesetzt werden.

Viele der erfindungsgemäss erhältlichen Verbindungen und ihre Verwendung sind bekannt und werden beschrieben z.B. in DE-OS 2 643 262, US-PS 4 127 652, EP-A 0 009 566, US-PS 4 325 948, US-PS 4 444 764 und US-PS 4 429 125.

Wie bereits oben dargelegt, ist es nach dem erfindungsgemässen Verfahren gemäss den Verfahrensstufen a) und b) möglich, die wertvollen Verbindungen der allgemeinen Formel (I) in glatten Reaktionen auf einfache Weise herzustellen, wobei hervorragende Gesamtausbeuten erhalten werden. Das erfindungsgemässe Verfahren a) und b) eröffnet in überraschender Weise durch die spezielle Kombination der Verfahrensschritte einen Weg, welcher eine bisher nicht erreichbare kostengünstige Herstellung der Verbindungen der allgemeinen Formel (I) erlaubt. Da die einzelnen Zwischenprodukte stabil sind und vor allem im Falle ihrer Isolierung über längere Zeit bevorratet werden können, erlaubt das erfindungsgemässe Verfahren darüber hinaus eine ausserordentliche Flexibilität in der Produktion, so dass bei rasch einsetzendem Bedarf der Endprodukte eine bedarfsgerechte Fertigung möglich ist, was insbesondere durch die klimatisch bedingten starken saisonellen Schwankungen auf dem Pflanzenschutzgebiet von sehr grosser Bedeutung sein kann.

Im folgenden sollen die erfindungsgemässen Verfahren (bzw. Verfahrensstufen) durch die nachstehenden Beispiele erläutert werden:

I. Verfahren zur Herstellung der Verbindungen der allgemeinen Formel (III) (Verfahrensstufe [a])

Beispiel I/1

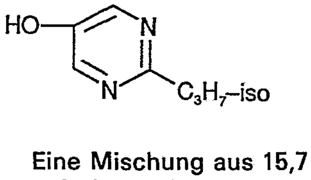

Eine Mischung aus 15,7 g (0,06 Mol) 5-Benzyloxy-4-chlor-1-isopropyl-pyrimidin, 8,1 g (0,08 Mol) Triethylamin, 1 g 10%iger Palladium/Kohle-Katalysator und 100 ml Ethanol werden bei 50°C unter 10 bar Wasserstoffdruck hydriert. Nach Ende der Reaktion wird vom Katalysator abgesaugt und im Vakuum eingedampft. Der Rückstand wird mit 25 ml Wasser verrieben; nach Kristallisation wird das Produkt abgesaugt und mit wenig Eiswasser nachgewaschen.

Man erhält so 6 g (72% der Theorie) 5-Hydroxy-2-iso-propyl-pyrimidin in Form farbloser Kristalle mit dem Schmelzpunkt 184°C.

Beispiel I/2

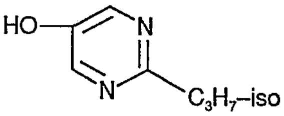

Eine Mischung aus 26,25 g (0,1 Mol) 5-Benzyloxy-4-chlor-1-isopropyl-pyrimidin, 200 ml Methylcyclohexan und 100 ml 2n Natronlauge wird in Gegenwart von 3 g Pd/Aktivkohle bei 100°C und 10 bis 20 bar Wasserstoffdruck hydriert. Dann wird der Katalysator abgesaugt, mit wenig Wasser nachgewaschen und im Filtrat die wässrige Phase abgetrennt. Sie wird im Vakuum kurz von anhaftenden Lösungsmittelresten befreit und mit Salzsäure auf pH 5 gebracht. Das ausgefallene Produkt wird bei 5°C abgesaugt und mit wenig Wasser nachgewaschen.

Man erhält auf diese Weise 10,8 g (78% der Theorie) 5-Hydroxy-2-isopropyl-pyrimidin in Form farbloser Kristalle mit dem Schmelzpunkt 182 bis 183°C.

Analog den Beispielen I/1 und I/2 werden die folgenden Verbindungen der Formel (III)

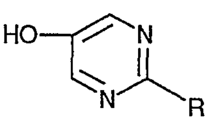 (III),

erhalten:

Tabelle 3

| Beispiel Nr. | R | Fp (°C) |
|---|---|---|
| I/3 | $C_3H_7$-n | 117 |
| I/4 | H | 216 |
| I/5 | $CH_3$ | 173 |
| I/6 | $N(CH_3)_2$ | 164 |
| I/7 | $C_2H_5$ | 149 |
| I/8 | 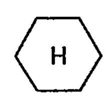 | 165 |
| I/9 | 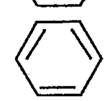 | 145 |

I. A) Herstellung der Ausgangsprodukte der allgemeinen Formel (II)

Beispiel IA/1

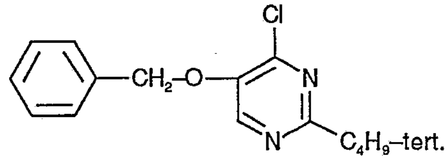

Zu einer Mischung aus 258 g (1 Mol) 5-Benzyloxy-2-tert.-butyl-4-hydroxy-pyrimidin und 300 ml Dimethylformamid lässt man unter Kühlen 169 g (1,1 Mol) Phosphoroxychlorid so zulaufen, dass die Reaktionstemperatur 50°C nicht übersteigt. Nach Ende der Zugabe wird noch 3 Stunden ohne Kühlung nachgerührt, dann wird das Gemisch auf 1 l Eiswasser gegeben und das ausgefallene Produkt abgesaugt. Es wird mit Wasser nachgewaschen und bei 30°C getrocknet.

Man erhält 257 g (93% der Theorie) 5-Benzyloxy-2-tert.-butyl-4-chlor-pyrimidin in Form eines farblosen Pulvers mit dem Schmelzpunkt 51°C.

Analog Beispiel (IA/1) können z.B. die folgenden Verbindungen der Formel (II) erhalten werden:

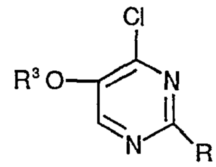

(II)

Tabelle 4

| Beispiel Nr. | R | $R^3$ | Fp (°C); Brechungsindex |
|---|---|---|---|
| IA/2 | $C_3H_7$-iso | 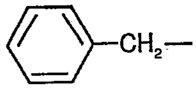 | 29 |
| IA/3 | H | 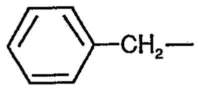 | 67 |
| IA/4 | 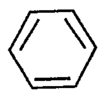 | 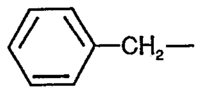 | 112 |
| IA/5 | $OCH_3$ | 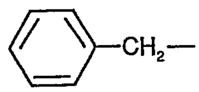 | 52 |
| IA/6 | $OC_3H_7$-iso | 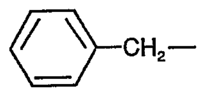 | $n_D^{21}$ : 1,5182 |

I. B) Herstellung der Ausgangsprodukte der allgemeinen Formel (V)

Beispiel IB/1

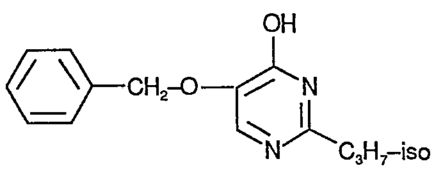

Eine Mischung aus 180 g (1 Mol) Benzyloxyessigsäuremethylester und 90 g (1,5 Mol) Ameisensäuremethylester wird bei maximal 25°C mit 81 g (1,5 Mol) Natriummethylat-Pulver versetzt. Man rührt 4 Stunden bei 20°C nach, gibt dann 200 ml Methanol, 0,6 Mol einer methanolischen Natriummethylatlösung und dann 122,5 g (1 Mol) Isobutyramidin-Hydrochlorid zu. Das Reaktionsgemisch kühlt sich dabei zunächst auf ca. 10°C bis 15°C ab und erwärmt sich dann langsam auf ca. 40°C. Nach 16 Stunden Rühren wird das Lösungsmittel im Vakuum abdestilliert, der Rückstand in ca. 1,5 l Wasser von 40°C gelöst und unter Kühlen bei ca. 30°C mit so viel konzentrierter Salzsäure versetzt, bis ein pH von 4 erreicht wird. Das Produkt wird bei 5°C abgesaugt und mit Wasser nachgewaschen.

Man erhält 200 g (82% der Theorie) 5-Benzyloxy-4-hydroxy-2-i-propyl-pyrimidin als farbloses Pulver mit dem Schmelzpunkt 198°C.

Analog Beispiel IB/1 werden z.B. die folgenden Verbindungen der Formel (V) hergestellt:

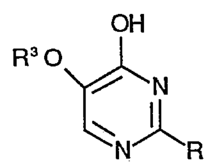

(V)

Tabelle 5

| Beispiel Nr. | R | $R^3$ | Fp (°C) |
|---|---|---|---|
| IB/2 | $C_4H_9$-tert. | 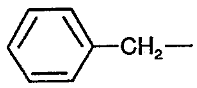 | 155 |
| IB/3 | H | 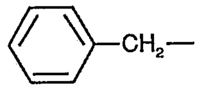 | 91 |
| IB/4 | 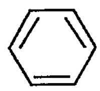 | 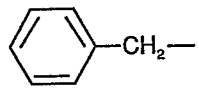 | 214 |
| IB/5 | $OCH_3$ | 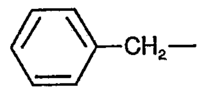 | 165 |
| IB/6 | $OC_3H_7$-i | 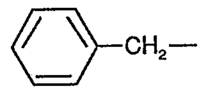 | 161 |

II. Verfahren zur Herstellung der Verbindungen der allgemeinen Formel (I) (Verfahrensstufe b)

Beispiel II/1

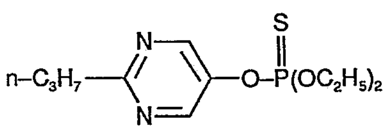

Ein Gemisch aus 300 ml Acetonitril, 13,8 g (0,1 Mol) 2-n-Propyl-5-hydroxy-pyrimidin, 20,7 g (0,15 Mol) Kaliumcarbonat und 18,8 g (0,1 Mol) O,O-Diethyl-thionophosphorsäurediesterchlorid wird 2 Stunden bei 45°C gerührt. Dann giesst man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an. Man erhält so 17,4 g (60% der Theorie) O,O-Diethyl-O-[2-n-propyl-pyrimidin(5)yl]-thionophosphorsäureester in Form eines braunen Öles mit dem Brechungsindex $n_D^{26}$: 1,4833.

In analoger Weise können die folgenden Verbindungen der Formel

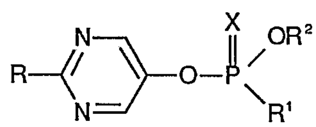 (I)

hergestellt werden:

| Beispiel Nr. | $R^2$ | $R^1$ | R | X | Ausbeute (% der Theorie) | Brechungsindex |
|---|---|---|---|---|---|---|
| II/2 | $C_3H_7$-iso | $CH_3$ | $C_3H_7$-iso | S | 74 | $n_D^{21}$: 1,5102 |
| II/3 | $CH_3$ | $OCH_3$ | $C_3H_7$-iso | S | 66 | $n_D^{24}$: 1,5080 |
| II/4 | $C_2H_5$ | $SC_3H_7$-n | $C_3H_7$-iso | S | 69 | $n_D^{26}$: 1,5284 |
| II/5 | $C_2H_5$ | 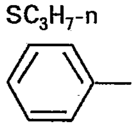 | $C_3H_7$-iso | S | 74 | $n_D^{26}$: 1,5570 |
| II/6 | $C_2H_5$ | $OC_2H_5$ | $C_3H_7$-iso | O | 82 | $n_D^{32}$: 1,4630 |
| II/7 | $C_2H_5$ | NH-$C_3H_7$-iso | $C_3H_7$-iso | S | 57 | $n_D^{32}$: 1,5057 |
| II/8 | $C_3H_7$-n | $OC_2H_5$ | $C_3H_7$-iso | S | 73 | $n_D^{32}$: 1,4929 |
| II/9 | $C_2H_5$ | $OC_2H_5$ | $CH_3$ | S | 92 | $n_D^{32}$: 1,4992 |
| II/10 | $C_2H_5$ | $C_2H_5$ | $CH_3$ | S | 80 | $n_D^{32}$: 1,5169 |
| II/11 | $C_2H_5$ | $OC_2H_5$ | 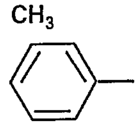 | S | 80 | $n_D^{32}$: 1,5643 |

| Beispiel Nr. | $R^2$ | $R^1$ | R | X | Ausbeute (% der Theorie) | Brechungs-index |
|---|---|---|---|---|---|---|
| II/12 | $C_2H_5$ | $C_2H_5$ | 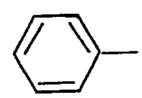 | S | 80 | $n_D^{32}$: 1,5827 |
| II/13 | $C_2H_5$ | $OC_2H_5$ | H | S | 72 | $n_D^{32}$: 1,5028 |
| II/14 | $C_2H_5$ | $OC_2H_5$ | $C_2H_5$ | S | 84 | $n_D^{20}$: 1,5014 |
| II/15 | $C_2H_5$ | $OC_2H_5$ | $C_4H_9$-n | S | 94 | $n_D^{21}$: 1,4958 |
| II/16 | $C_2H_5$ | $OC_2H_5$ | $C_4H_9$-tert. | S | 86 | $n_D^{26}$: 1,4902 |

Beispiel II/17

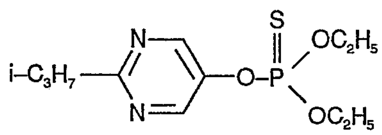

Eine Mischung aus 180 g (1 Mol) Benzyloxyessigsäuremethylester und 90 g (1,5 Mol) Ameisensäuremethylester wird bei maximal 25°C mit 81 g (1,5 Mol) Natriummethylat-Pulver versetzt. Man rührt 4 Stunden bei 20°C nach, gibt dann 200 ml Methanol, 0,6 Mol einer methanolischen Natriummethylatlösung und dann 122,5 g (1 Mol) Isobutyramidin-Hydrochlorid zu. Das Reaktionsgemisch kühlt sich dabei zunächst auf ca. 10°C bis 15°C ab und erwärmt sich dann langsam auf ca. 40°C. Nach 16 Stunden rühren wird das Lösungsmittel im Vakuum abdestilliert, der Rückstand in ca. 1,5 l Wasser von 40°C gelöst und unter Kühlen bei ca. 30°C mit so viel konzentrierter Salzsäure versetzt, bis ein pH von 4 erreicht wird. Das Produkt wird bei 5°C abgesaugt und mit Wasser nachgewaschen. Nach dem Trocknen löst man die Verbindung in 600 ml Chloroform, destilliert ca. 50 ml Lösungsmittel ab und tropft dann unter Sieden 138,6 g (0,9 Mol) Phosphoroxychlorid zu und kocht die Mischung 20 Stunden unter Rückfluss. Nach Abkühlen auf 0°C tropft man 160 ml konzentrierte Ammoniak-Lösung zu, trennt die organische Phase ab und destilliert nach dem Trocknen über Natriumsulfat das Lösungsmittel im Vakuum ab.

Der Rückstand wird zu einem Gemisch aus 800 ml Methylcyclohexan und 750 ml 2n-Natronlauge gegeben und in Gegenwart von 15 g Pd/Aktivkohle bei 100°C und 10 bis 20 bar Wasserstoffdruck hydriert. Dann wird der Katalysator abgesaugt, mit wenig Wasser nachgewaschen und im Filtrat die wässrige Phase abgetrennt. Sie wird im Vakuum kurz von anhaftenden Lösungsmittelresten befreit und mit Salzsäure auf pH 5 gebracht. Das ausgefallene Produkt wird bei 5°C abgesaugt und mit wenig Wasser nachgewaschen. Man trocknet es an der Luft, gibt es zu einer Mischung aus 300 ml Acetonitril, 124,2 g (0,9 Mol) Kaliumcarbonat und 113,1 g (0,6 Mol) 0,0-Diethylthiophosphorsäurediesterchlorid und rührt 2 Stunden bei 45°C nach. Dann destilliert man das Lösungsmittel im Vakuum ab, löst den Rückstand in 400 ml Toluol und wäscht die Lösung zweimal mit je 300 ml Wasser. Die Toluollösung wird über Natriumsulfat getrocknet und im Vakuum eingedampft. Den Rückstand destilliert man im Hochvakuum an. Man erhält so 170,5 g (59% der Theorie) 0,0-Diethyl-0-[2-isopropyl-pyrimidin(5)yl]-thionophosphorsäureester in Form eines braunen Öles mit dem Brechungsindex $n_D^{21}$: 1,4970.

Beispiel II/18

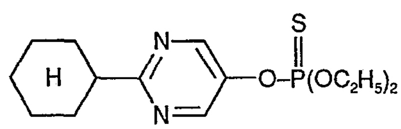

Ein Gemisch aus 300 ml Acetonitril, 17,8 g (0,1 Mol) 2-Cyclohexyl-5-hydroxy-pyrimidin, 20,7 g (0,15 Mol) Kaliumcarbonat und 18,8 g (0,1 Mol) O,O-Diethylthionophosphorsäurediesterchlorid wird 2 Stunden bei 45°C gerührt. Dann giesst man das Reaktionsgemisch in 400 ml Toluol und wäscht es zweimal mit je 300 ml Wasser. Die Toluollösung wird über Natriumsulfat getrocknet und im Valuum eingedampft. Den Rückstand destilliert man im Hochvakuum an. Man erhält so 21,7 g (66% der Theorie) O,O-Diethyl-0-[2-cyclohexyl-pyrimidin(5)yl]-thionophosphorsäureester in Form eines braunen Öles mit dem Brechungsindex $n_D^{23}$: 1,5158.

In analoger Weise können die folgenden Verbindungen der Formel

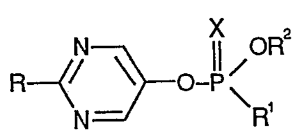

hergestellt werden:

| Beispiel Nr. | $R^2$ | $R^1$ | R | X | Ausbeute (% der Theorie) | Physikal. Daten (Brechungsindex; Schmelzpunkt °C) |
|---|---|---|---|---|---|---|
| II/19 | $C_2H_5$ | NH-$C_3H_7$-iso | 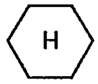 | S | 51 | $n_D^{23}$: 1,5246 |
| II/20 | $CH_3$ | $OCH_3$ | 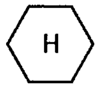 | S | 64 | $n_D^{23}$: 1,5287 |
| II/21 | $C_2H_5$ | $OC_2H_5$ | 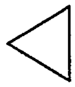 | S | 78 | $n_D^{24}$: 1,5142 |
| II/22 | $C_2H_5$ | NH-$C_3H_7$-iso | 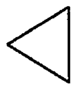 | S | 62 | 49 |
| II/23 | $CH_3$ | $OCH_3$ | 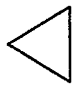 | S | 43 | $n_D^{24}$: 1,5390 |
| II/24 | $C_3H_7$-n | $OC_2H_5$ | 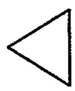 | S | 71 | $n_D^{25}$: 1,5128 |
| II/25 | $C_2H_5$ | NH-$C_2H_5$ | 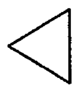 | S | 74 | $n_D^{26}$: 1,5310 |
| II/26 | $C_2H_5$ | $OC_2H_5$ | 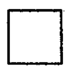 | S | | |
| II/27 | $C_2H_5$ | $OC_2H_5$ | 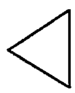 | S | | |
| II/28 | $C_2H_5$ | $OC_2H_5$ | 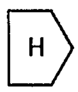 | S | 80 | $n_D^{23}$: 1,5164 |
| II/29 | $C_2H_5$ | $OC_3H_7$-n | 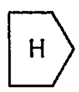 | S | | |
| II/30 | $C_2H_5$ | $CH_3$ | 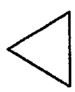 | S | 72 | $n_D^{25}$: 1,5428 |
| II/31 | $C_2H_5$ | $OC_2H_5$ | 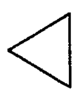 | O | | |
| II/32 | $C_2H_5$ | NH-$C_3H_7$-iso | 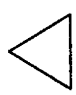 | O | | |
| II/33 | $C_2H_5$ | 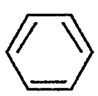 | 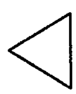 | S | 74 | $n_D^{25}$: 1,5815 |
| II/34 | $C_2H_5$ | $SC_3H_7$-n | 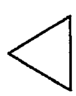 | S | | |
| II/35 | $C_2H_5$ | 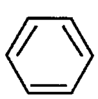 | 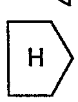 | S | | |
| II/36 | $C_2H_5$ | NH-$C_2H_5$ | 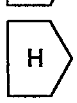 | S | 66 | $n_D^{23}$: 1,5329 |
| II/37 | $C_2H_5$ | $SC_3H_7$ | 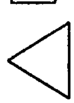 | O | | |

| Beispiel Nr. | $R^2$ | $R^1$ | R | X | Ausbeute (% der Theorie) | Physikal. Daten (Brechungsindex; Schmelzpunkt °C) |
|---|---|---|---|---|---|---|
| II/38 | $C_2H_5$ | $C_2H_5$ | 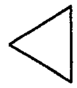 | S | | |
| II/39 | $CH_3$ | $C_2H_5$ | 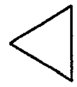 | S | | |
| II/40 | $C_3H_7$-iso | $CH_3$ | 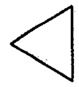 | S | 67 | $n_D^{26}$: 1,5233 |
| II/41 | $CH_3$ | NH-$C_3H_7$-iso | 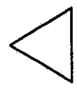 | S | | |
| II/42 | $CH_3$ | NH-$CH_3$ | 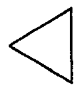 | S | 66 | $n_D^{26}$: 1,5460 |
| II/43 | $C_2H_5$ | NH-$CH_3$ | 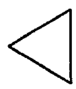 | S | | |
| II/44 | $CH_3$ | NH-$C_2H_5$ | 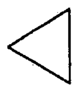 | S | | |
| II/45 | $C_2H_5$ | NH-$C_3H_7$-iso | 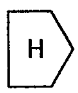 | S | 55 | $n_D^{26}$: 1,524 |
| II/46 | $C_2H_5$ | $OC_2H_5$ | 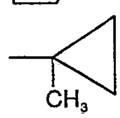 | S | | |

Beispiel II/47

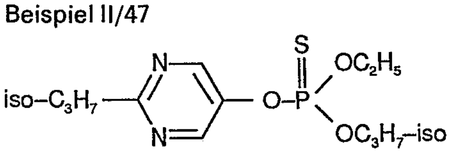

Ein Gemisch aus 300 ml Acetonitril, 13,8 g (0,1 Mol) 5-Hydroxy-2-iso-propyl-pyrimidin, 20,7 g (0,15 Mol) Kaliumcarbonat und 20,2 g (0,1 Mol) O-Ethyl-O-iso-propyl-thionophosphorsäurediester-chlorid wird 2 Stunden bei 45°C gerührt. Dann giesst man das Reaktionsgemisch in 400 ml Toluol und wäscht es zweimal mit je 300 ml Wasser. Die Toluollösung wird über Natriumsulfat getrocknet und im Vakuum eingedampft. Den Rückstand destilliert man im Hochvakuum an.

Man erhält so 28 g (92% der Theorie) O-Ethyl-O-iso-propyl-O-(2-iso-propyl-pyrimidin-5-yl)-thionophosphorsäureester in Form eines gelben Öles mit dem Brechungsindex $n_D^{23}$ : 1,4910.

In analoger Weise können die folgenden Verbindungen der Formel

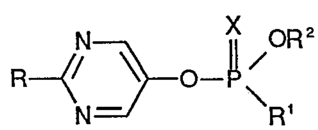

(I)

hergestellt werden:

| Beispiel Nr. | R | $R^2$ | $R^1$ | Brechungsindex |
|---|---|---|---|---|
| II/48 | -$C_3H_7$-iso | -$OC_3H_7$-iso | -$C_3H_7$-iso | $n_D^{20}$: 1,4869 |
| II/49 | -$C_4H_9$-tert. | -$C_2H_5$ | -$OC_3H_7$-iso | $n_D^{20}$: 1,4917 |
| II/50 | -$C_3H_7$-iso | -$C_2H_5$ | -$OC_4H_9$-sec. | $n_D^{20}$: 1,4960 |
| II/51 | -$C_4H_9$-tert. | -$C_2H_5$ | -$OC_4H_9$-sec. | $n_D^{22}$: 1,4935 |
| II/52 | -$C_4H_9$-tert. | -$C_3H_7$-iso | $OC_3H_7$-iso | $n_D^{22}$: 1,4857 |
| II/53 | 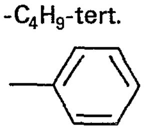 | -$C_2H_5$ | -$OC_3H_7$-iso | $n_D^{22}$: 1,5516 |

| Beispiel Nr. | R | $R^2$ | $R^1$ | Brechungsindex |
|---|---|---|---|---|
| II/54 | $-C_4H_9$-tert. | $-C_2H_5$ | $-NHC_2H_5$ | $n_D^{21}$: 1,5100 |
| II/55 | 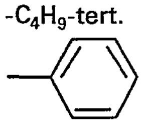 | $-C_2H_5$ | $-OC_4H_9$-sec. | |
| II/56 | 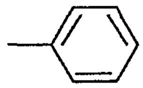 | $-C_3H_7$-iso | $-OC_3H_7$-iso | |
| II/57 | $-C_3H_7$-iso | $-C_3H_7$-n | $-OC_3H_7$-n | $n_D^{23}$: 1,4915 |

**Patentansprüche**

1. Verfahren zur Herstellung von Verbindungen der allgemeinen Formel (I)

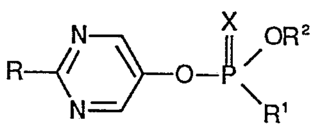

(I)

in welcher
R für Wasserstoff, Alkoxy, Alkylamino, Dialkylamino oder für gegebenenfalls substituierte Reste aus der Reihe Alkyl, Cycloalkyl und Aryl steht,
$R^1$ für gegebenenfalls substituierte Reste aus der Reihe Alkyl, Alkoxy, Alkylthio, Mono- oder Dialkylamino und Phenyl steht,
$R^2$ für gegebenenfalls substituiertes Alkyl steht und
X für Sauerstoff oder Schwefel steht, dadurch gekennzeichnet, dass man

a) Verbindungen der allgemeinen Formel (II)

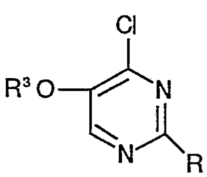

(II)

in welcher
R die oben angegebene Bedeutung hat, und
$R^3$ für gegebenenfalls substituiertes Benzyl steht,
mit Wasserstoff in Gegenwart von Hydrierungskatalysatoren, in Gegenwart von Säureakzeptoren und in Gegenwart von Verdünnungsmitteln, bei Temperaturen zwischen 20°C und 150°C zu den Verbindungen der allgemeinen Formel (III)

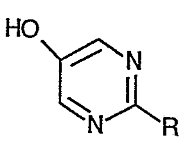

(III)

in welcher
R die oben angegebene Bedeutung hat, umsetzt und anschliessend

b) die Verbindungen der allgemeinen Formel (III), gegebenenfalls nach ihrer Isolierung, mit Verbindungen der allgemeinen Formel (IV)

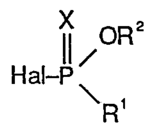

(IV)

in welcher
Hal für Halogen steht und
X, $R^1$ und $R^2$ die oben angegebene Bedeutung haben,
gegebenenfalls in Gegenwart eines Säurebindemittels und gegebenenfalls in Gegenwart eines Lösungsmittels, umsetzt und die Verbindungen der allgemeinen Formel (I) isoliert.

2. Verfahren gemäss Anspruch 1, dadurch gekennzeichnet, dass R für $C_1$-$C_4$-Alkyl, $C_3$-$C_6$-Cycloalkyl oder Phenyl, $R^1$ fpr $C_1$-$C_4$-Alkoxy, $R^2$ für $C_1$-$C_4$-Alkyl und X für Schwefel stehen.

3. Verfahren gemäss Anspruch 1, dadurch gekennzeichnet, dass R für $C_1$-$C_4$-Alkyl, $R^1$ für $C_1$-$C_4$-Alkoxy, $R^2$ für $C_1$-$C_4$-Alkyl und X für Schwefel stehen.

4. Verfahren zur Herstellung von 5-Hydroxypyrimidinen der allgemeinen Formel (III)

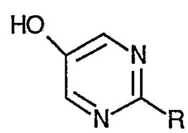

(III)

in welcher
R für Wasserstoff, Alkoxy, Alkylamino, Dialkylamino oder für gegebenenfalls substituierte Reste aus der Reihe Alkyl, Cycloalkyl und Aryl steht,
dadurch gekennzeichnet, dass man substituierte 4-Chlor-pyrimidin-Derivate der allgemeinen Formel (II)

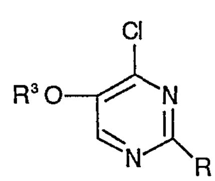

(II)

in welcher
R die oben angegebenen Bedeutungen hat und
$R^3$ für gegebenenfalls substituiertes Benzyl steht,

mit Wasserstoff in Gegenwart von Hydrierungskatalysatoren, in Gegenwart von Säureakzeptoren und in Gegenwart von Verdünnungsmitteln, bei Temperaturen zwischen 20°C und 150°C umsetzt und gewünschtenfalls die Verbindungen der allgemeinen Formel (III) isoliert.

5. Verfahren gemäss Anspruch 4, wobei R für Wasserstoff, Alkoxy mit 1 bis 12 Kohlenstoffatomen, für Mono- oder Dialkylamino mit jeweils 1 bis 6 Kohlenstoffatomen im Alkylteil, für gegebenenfalls durch $C_1$–$C_4$-Alkoxy oder $C_1$–$C_4$-Alkylsulfonyl substituiertes Alkyl mit 1 bis 12 Kohlenstoffatomen, für gegebenenfalls durch $C_1$–$C_4$-Alkyl substituiertes Cycloalkyl mit 3 bis 8 Kohlenstoffatomen und für gegebenenfalls durch $C_1$–$C_4$-Alkyl, $C_1$–$C_4$-Alkoxy oder $C_1$–$C_4$-Alkylsulfonyl substituiertes Aryl mit 6 bis 10 Kohlenstoffatomen steht.

6. Verfahren gemäss Anspruch 4, wobei R für Wasserstoff, Alkoxy mit 1 bis 6 Kohlenstoffatomen, Mono- oder Dialkylamino mit jeweils 1 bis 4 Kohlenstoffatomen im Alkylteil oder für gegebenenfalls durch Methoxy, Ethoxy, Methylsulfonyl oder Ethylsulfonyl substituiertes Alkyl mit 1 bis 6 Kohlenstoffatomen, für gegebenenfalls durch Methyl oder Ethyl substituiertes Cycloalkyl mit 3 bis 6 Kohlenstoffatomen und für gegebenenfalls durch Methyl, Ethyl, Methoxy, Ethoxy, Methylsulfonyl oder Ethylsulfonyl substituiertes Phenyl steht.

7. Verfahren gemäss Anspruch 4, wobei R für $C_1$–$C_4$-Alkyl, $C_3$–$C_6$-Cycloalkyl oder Phenyl steht.

8. Verfahren gemäss Anspruch 4, wobei R für Methyl, Ethyl, Isopropyl und t.-Butyl steht.

**Claims**

1. Process for the preparation of compounds of the general formula (I)

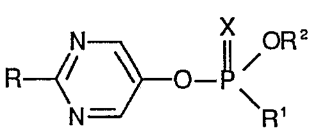

(I)

in which

R represents hydrogen, alkoxy, alkylamino, dialkylamino or optionally substituted radicals from the group comprising alkyl, cycloalkyl and aryl,

$R^1$ represents optionally substituted radicals from the group comprising alkyl, alkoxy, alkylthio, monoalkylamino or dialkylamino and phenyl,

$R^2$ represents optionally substituted alkyl, and X represents oxygen or sulphur,

characterised in that

a) compunds of the general formula (II)

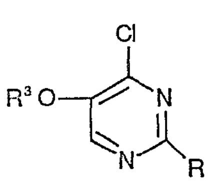

(II)

in which

R has the abovementioned meaning, and

$R^3$ represents optionally substituted benzyl,

are reacted with hydrogen in the presence of hydrogenation catalysts, in the presence of acid acceptors and in the presence of diluents, at temperatures between 20°C and 150°C, to give the compounds of the general formula (III)

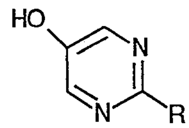

(III)

in which

R has the abovementioned meaning, and then

b) the compounds of the general formula (III), where appropriate after their isolation, are reacted with compounds of the general formula (IV)

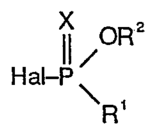

(IV)

in which

Hal represents halogen, and

X, $R^1$ and $R^2$ have the abovementioned meaning,

where appropriate in the presence of an acid-binding agent and, where appropriate, in the presence of a solvent, and the compounds of the general formula (I) are isolated.

2. Process according to claim 1, characterised in that R represents $C_1$–$C_4$-alkyl, $C_3$–$C_6$-cycloalkyl or phenyl, $R^1$ represents $C_1$–$C_4$-alkoxy, $R^2$ represents $C_1$–$C_4$-alkyl and X represents sulphur.

3. Process according to claim 1, characterised in that R represents $C_1$–$C_4$-alkyl, $R^1$ represents $C_1$–$C_4$-alkoxy, $R^2$ represents $C_1$–$C_4$-alkyl and X represents sulphur.

4. Process for the preparation of 5-hydroxypyrimidines of the general formula (III)

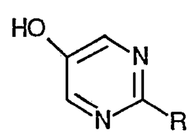

(III)

in which

R represents hydrogen, alkoxy, alkylamino, dialkylamino or optionally substituted radicals from the group comprising alkyl, cycloalkyl and aryl,

characterised in that substituted 4-chloropyrimidine derivatives of the general formula (II)

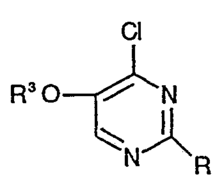

(II)

in which

R has the abovementioned meanings, and

$R^3$ represents optionally substituted benzyl, are reacted with hydrogen in the presence of hydrogenation catalysts, in the presence of acid acceptors and in the presence of diluents, at temperatures between 20°C and 150°C, and, if desired, the compounds of the general formula (III) are isolated.

5. Process according to Claim 4, where R represents hydrogen, alkoxy having 1 to 12 carbon atoms, monoalkylamino or dialkylamino each having 1 to 6 carbon atoms in the alkyl moiety, alkyl which has 1 to 12 carbon atoms and is optionally substituted by $C_1$–$C_4$-alkoxy or $C_1$–$C_4$-alkylsulphonyl, cycloalkyl which has 3 to 8 carbon atoms and is optionally substituted by $C_1$–$C_4$-alkyl, and aryl which has 6 to 10 carbon atoms and is optionally substituted by $C_1$–$C_4$-alkyl, $C_1$–$C_4$-alkoxy or $C_1$–$C_4$-alkylsulphonyl.

6. Process according to Claim 4, where R represents hydrogen, alkoxy having 1 to 6 carbon atoms, monoalkylamino or dialkylamino each having 1 to 4 carbon atoms in the alkyl moiety, or alkyl which has 1 to 6 carbon atoms and is optionally substituted by methoxy, ethoxy, methylsulphonyl or ethylsulphonyl, cycloalkyl which has 3 to 6 carbon atoms and is optionally substituted by methyl or ethyl, and phenyl which is optionally substituted by methyl, ethyl, methoxy, ethoxy, methylsulphonyl or ethylsulphonyl.

7. Process according to claim 4, where R represents $C_1$–$C_4$-alkyl, $C_3$–$C_6$-cycloalkyl or phenyl.

8. Process according to Claim 4, where R represents methyl, ethyl, isopropyl and t.-butyl.

**Revendications**

1. Procédé de production de composés de formule générale (I)

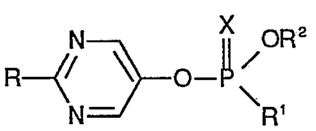 (I)

dans laquelle
R représente l'hydrogène, un groupe alkoxy, alkylamino, dialkylamino ou des restes éventuellement substitués de la série alkyle, cycloalkyle et aryle,
$R^1$ représente des restes éventuellement substitués de la série alkyle, alkoxy, alkylthio, mono- ou dialkylamino et phényle,
$R^2$ représente un groupe alkyle éventuellement substitué et
X est l'oxygène ou le soufre,
caractérisé en ce que:

a) on fait réagir des composés de formule générale (II)

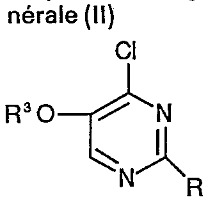 (II)

dans laquelle
R a la définition indiquée ci-dessus, et
$R^3$ est un groupe benzyle éventuellement substitué,
avec de l'hydrogène en présence de catalyseurs d'hydrogénation, en présence d'accepteurs d'acides et en présence de diluants, à des températures comprises entre 20 et 150°C pour former les composés de formule générale (III)

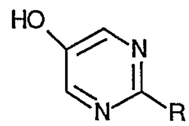 (III)

dans laquelle
R a la définition indiquée ci-dessus, puis

b) on fait réagir les composés de formule générale (III), éventuellement après leur isolement, avec des composés de formule générale (IV)

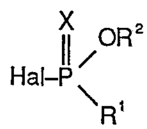 (IV)

dans laquelle
Hal représente un halogène et
X, $R^1$ et $R^2$ ont la définition indiquée ci-dessus
éventuellement en présence d'un accepteur d'acide et le cas échéant en présence d'un solvant,et on isole les composés de formule générale (I).

2. Procédé suivant la revendication 1, caractérisé en ce que R est un groupe alkyle en $C_1$ à $C_4$, cycloalkyle en $C_3$ à $C_6$ ou phényle, $R^1$ est un groupe alkoxy en $C_1$ à $C_4$, $R^2$ est un groupe alkyle en $C_1$ à $C_4$ et X est le soufre.

3. Procédé suivant la revendication 1, caractérisé en ce que R est un groupe alkyle en $C_1$ à $C_4$, $R^1$ est un groupe alkoxy en $C_1$ à $C_4$, $R^2$ est un groupe alkyle en $C_1$ à $C_4$ et X est le soufre.

4. Procédé de préparation de 5-hydroxypyrimidines de formule générale (III)

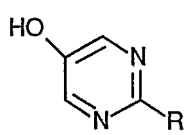 (III)

dans laquelle
R représente l'hydrogène, un groupe alkoxy, alkylamino, dialkylamino ou des restes éventuellement substitués de la série alkyle, cycloalkyle et aryle,
caractérisé en ce qu'on fait réagir des dérivés substitués de 4-chloropyrimidine de formule générale (II)

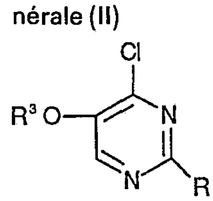 (II)

dans laquelle
R a les définitions indiquées ci-dessus et
$R^3$ est un groupe benzyle éventuellement substitué, avec de l'hydrogène en présence de catalyseurs d'hydrogénation, en présence d'accepteurs d'acides et en présence de diluants, à des températures comprises entre 20 et 150°C et on isole, si on le désire, les composés de formule générale (III).

5. Procédé suivant la revendication 4, dans lequel R représente l'hydrogène, un groupe alkoxy ayant 1 à 12 atomes de carbone, un groupe mono- ou dialkylamino ayant chacun 1 à 6 atomes de carbone dans la partie alkyle, un groupe alkyle de 1 à 12 atomes de carbone éventuellement substitué par un radical alkoxy en $C_1$ à $C_4$ ou alkylsulfonyle en $C_1$ à $C_4$, un groupe cycloalkyle de 3 à 8 atomes de carbone éventuellement substitué par un radical alkyle en $C_1$ à $C_4$ et un groupe aryle de 6 à 10 atomes de carbone éventuellement substitué par un radical alkyle en $C_1$ à $C_4$, alkoxy en $C_1$ à $C_4$ ou alkysulfonyle en $C_1$ à $C_4$.

6. Procédé suivant la revendication 1, dans lequel
R représente l'hydrogène, un groupe alkoxy ayant 1 à 6 atomes de carbone, un groupe mono- ou dialkylamino ayant chacun 1 à 4 atomes de carbone dans la partie alkyle ou un groupe alkyle de 1 à 6 atomes de carbone éventuellement substitué par un radical méthoxy, éthoxy, méthylsulfonyle ou éthylsulfonyle, un groupe cycloalkyle de 3 à 6 atomes de carbone éventuellement substitué par un radical méthyle ou éthyle et un groupe phényle éventuellement substitué par un radical méthyle, éthyle, méthoxy, éthoxy, méthylsulfonyle ou éthylsulfonyle.

7. Procédé suivant la revendication 4, dans lequel R est un groupe alkyle en $C_1$ à $C_4$, cycloalkyle en $C_3$ à $C_6$ ou phényle.

8. Procédé suivant la revendication 4, dans lequel R est un groupe méthyle, éthyle, isopropyle ou tertio-butyle.